# EUROPEAN PATENT APPLICATION

(11) **EP 3 144 016 A1**
(43) Date of publication of application: **22.03.2017**
(21) Application number: 15789667.1
(22) Date of filing: 05.05.2015
(51) Int. Cl.: A61L 27/12, A61L 24/02, A61F 2/28

(54) **INORGANIC, INJECTABLE AND THERMOSENSITIVE CEMENT FOR BONE RECONSTRUCTION: PREPARATION AND USE**

(30) Priority: 05.05.2014 ES 201430644
(71) Applicant: Universitat Politècnica De Catalunya, 08034 Barcelona (ES)
(72) Inventor: GINEBRA MOLINS, Maria Pau, 08034 Barcelona (ES); MONTUFAR JIMENEZ, Edgar Benjamin, 08034 Barcelona (ES); MAAZOUZ, Yassine, 08034 Barcelona (ES); PASTORINO, David, 08034 Barcelona (ES)
(74) Representative: Juncosa Miro, Jaime
(86) International application number: PCT/ES2015/070361
(87) International publication number: WO 2015/169992

(57) **Abstract**

Inorganic, injectable and thermosensitive cement for biomedical applications: preparation and use

The preparation of a thermosensitive, injectable cement is proposed with clinical applications in bone surgery and minimally invasive dentistry, based on self-setting mixtures of magnesium oxide, calcium, sodium or ammonium phosphates, calcium carbonates, calcium sulphates or other inorganic salts with a poloxamer hydrogel. This cement exhibits a thermosensitive effect as one of its main properties. It also exhibits high cohesion in contact with fluids at physiological temperature, accompanied by a temperature-related decrease in the force necessary for its injection.

Indications are given as to the use of the cement for bone and dental applications, as well as for the filling of bone defects. The cement is especially indicated in cases where a minimally invasive use was necessary.

## Description

### Field of the invention

The present invention relates to biomaterials for regenerating hard tissues: bones and teeth. These materials may be prepared in the form of pellets, cements, coatings, dense or porous ceramics, etc. Among other applications, they may be applied to fill in bone cavities, cranial defects or cavities left after the removal of a tumour, to fill in gaps after a multiple fracture, to fill in sockets after a tooth is extracted, to increase the amount of bone before placing a dental implant, to fix screws when placing metal plates, to stabilise bone fractures or to coat prostheses and implants.

An entirely injectable inorganic cement is hereby proposed, whereby it is especially suitable for minimally surgically invasive medical and veterinary procedures. However, it also may be used in traditional surgery. In particular, the cement exhibits thermosensitive properties; i.e., certain properties, such as cohesion, injection force and set time, can be adjusted by controlling the paste temperature. On the other hand, the final product of the setting reaction, depending on the starting composition, may comprise calcium phosphates, such as hydroxyapatite, calcium-deficient hydroxyapatite, brushite or monetite, having great compositional and structural similarity to the mineral fraction of bones and teeth, or other inorganic salts, such as magnesium phosphates, calcium carbonates or calcium sulphates, having mechanical strength suitable for bone and dental applications. The cement may likewise act as a support for a controlled-release drug delivery system and for cellular ingrowth in tissue engineering scaffolds. The present invention also relates to methods for obtaining said biomaterials.

The current invention also relates to a method for the preparation of said cement and to the use of said cement for bone and dental applications.

### State of the prior art

Since the mid-eighties, the scientific community has made important advances in the field of the design and manufacture of new materials for the substitution and regeneration of mineralised biological tissues. One type of materials that has been extensively studied are materials based on inorganic salts, such as calcium or magnesium phosphates, calcium sulphates or calcium carbonates. From among the different ways in which these materials can be presented, the cements deserve especial attention, due to certain intrinsic characteristics, such as their mouldability and perfect adaptation to the bone cavity, the possibility of injecting them using minimally invasive techniques and their ability to harden once they have been implanted within the body. It is noteworthy that, in the case of calcium phosphate cements, hydroxyapatite can be obtained as the reaction product, which exhibits many similarities with the natural inorganic bone component, such as its chemical composition and the nanometric size of its crystals.

Recent developments in minimally invasive surgery have reduced the risk incurred by the patient during the surgical procedure and the relevant hospital charges. This new surgical technique requires new fully injectable bone regeneration materials, capable of hardening *in situ* and of regenerating the damaged bone. In spite of the recognised ability of inorganic cements to fill and/or satisfactorily regenerate damaged bone, they also have certain limitations. First of all, the injectability of inorganic cement pastes is limited. Secondly, the setting time of injectable cements tends to be higher than that of non-injectable cements. Lastly, the third limitation is the low mechanical strength of injectable cements.

The injectability of the inorganic cements can be improved by increasing the liquid-powder mixing ratio. This alternative, however, delays the setting of the cement, facilitates the disintegration of the paste upon contact with a liquid medium and considerably reduces the mechanical strength. Another alternative is the addition of a polymer, usually a hydrogel, in the liquid fraction of the cement. In this case, the injectability and the cohesion of the pastes increase considerably, but the force required to inject the paste also increases significantly. Besides, the addition of hydrogel produces side effects such as the increase in setting time and the reduction of the mechanical strength. Hydrogels used to improve the injectability of inorganic cements for biomedical use are mostly of natural origin, such as cellulose and its derivatives, chitosan, collagen, gelatin, sodium alginate or hyaluronic acid. Precisely because of their natural origin, special care must be taken to avoid possible immunological rejections or changes in properties among the different batches of the hydrogel.

Poloxamers are one family of polymers of synthetic origin and, therefore, lacking associated problems such as immunological rejection or the variability of their properties. Poloxamers are three-block copolymers composed of a central hydrophobic poly(propylene oxide) portion flanked by two hydrophilic side poly(ethylene oxide) portions [1]. Depending on the length of the three chains, there are different types of poloxamers. In general, poloxamers are considered non-ionic surfactants. As they increase their concentration in water, the molecules of this copolymer associate to form micelles, and if the concentration continues to increase, they are able to form hydrogels. The concentration at which the hydrogel is formed depends on the type of poloxamer and on the temperature. In general, at low temperatures poloxamer solutions have low viscosity and they behave like liquids, whereas at high temperatures their viscosity increases and they behave as hydrogels. Poloxamers are biocompatible, do not significantly alter biological functions and are excreted via the kidneys. For these reasons, poloxamers are used in a wide variety of biomedical applications, such as systems for the controlled release of drugs, excipients in medications or temporary embolisers, among many others [1].

In an area very distant from the development of inorganic cements for biomedical use in minimally invasive surgery, poloxamers have been used, because of their rheological characteristics, in direct three-dimensional printing processes to form ceramic inks for the manufacture by robotic additive moulding of scaffolds for tissue engineering. In particular, poloxamers have been mixed with non-reactive calcium phosphates, such as bioactive glass, hydroxyapatite, beta tricalcium phosphate or mixtures of the last two [2]. That fact that these inks do not have the ability to set like a cement has three important consequences. The first one is that the time elapsed between the mixture and the printing of the inks does not affect the force required for the extrusion. The second one is that consolidation of manufactured structures is carried out by sintering between 700 and 1400°C, which implies that, although the compositional similarity with the mineral fraction of bones and teeth is maintained, the microstructural similarity is lost, producing materials of difficult *in vivo* reabsorption. Lastly, the manufactured scaffolds are rigid and have predefined shapes and sizes, so they cannot be implanted by means of non-invasive surgical techniques.

Because of their ability to gel as temperature increases, their solubility in aqueous media, their biocompatibility and their ability to be excreted via the kidneys, poloxamer hydrogels are used as a temporary emboliser in the field of cardiovascular medicine [3]. However, the thermosensitive properties of poloxamers have not been exploited in orthopaedic or oral surgery, let alone in formulations of inorganic cements for biomedical use. The use of poloxamers in calcium phosphate cements has been limited to their surfactant character to avoid the aggregation of the powder of the cement [4] or for the formation of foams [5].

### Brief explanation of the invention

In contrast to the aforementioned works and patents, the current invention presents an inorganic/organic hybrid cement for biomedical use whose properties, such as cohesion, set time and injectability, can be adjusted by controlling the temperature. The paste is based on a mixture of self-setting inorganic salts with water or aqueous solutions, with or without the admixture of additives that will accelerate or delay the setting of the cement, and the addition of one poloxamer in the liquid fraction or in the solid fraction, so as to provide the desired thermosensitive character. The applications of the cement are both traditional and minimally invasive clinical, bone or dental surgical applications. Said cement exhibits several intrinsic properties that make it of special interest. The first characteristic to be highlighted in the material is that, regardless of the initial temperature of the paste, the cement has cohesion when it is injected into a medium whose temperature is 37°C. This characteristic makes it possible for the cement to set and harden *in situ* without disintegrating or undergoing erosion. The second relevant characteristic is that the pastes of cement with poloxamer are fully injectable applying forces inferior to those used to inject acrylic cements, and do not exhibit fraction separation. The most important point to note is that the injection force diminishes with the decrease in the temperature of the paste. The injection force reaches a minimum and remains constant below 15°C. In addition, the pastes exhibit short setting times compared to other cements incorporating hydrogels to improve injectability. The cement hardens either through the formation and crosslinking of crystals of the fractions that occur in the setting reaction, or through the formation of an amorphous fraction, which result in an increase in mechanical strength.

Together, all these characteristics make the cement herein presented ideal for minimally invasive surgery wherever regenerating bone or dental lesions is required, but it is also suitable for traditional surgery, both in medicine and veterinary medicine. Adjusting the injection force allows the surgeon to inject the material as accurately as possible and with a minimum of effort, whereas the cohesion of the paste will ensure the success of the surgical procedure, getting the paste to set in the desired position, achieving a reasonable mechanical strength, similar to that of trabecular bone. Lastly, the setting product of the cement is fully biocompatible, and in some specific cases it can get to be osteoconductive and resorbable, which ensures the regeneration of damaged tissue.

The composition of the material has been optimised in order to guarantee the aspects mentioned in the previous paragraph. The preparation of the cement requires a solid fraction or powder, composed of one or several inorganic salts, and a liquid fraction, composed mainly of water or some biocompatible solvent wherein the poloxamer can be dissolved. The mixture of both fractions can be carried out manually or in a high-energy mixer, at a temperature below or equal to room temperature. The temperature of the solid fraction and/or the liquid fraction at the time of mixing can be equal to or below 25°C, preferably below 12°C. The paste obtained can be directly implanted in the patient by traditional surgery or it can be placed in a syringe in order to be implanted by minimally invasive surgery. Alternatively, the paste may be placed in a syringe or a similar device for its subsequent freezing, preferably at temperatures equal to or below -20°C, for storage, and it is subsequently thawed preferably at less than 12°C for use in clinical application. Once implanted, the cement paste will harden *in situ* due to its good cohesion at physiological temperature, and it will produce a rigid body with compressive strength similar to that of trabecular bone.

It is important to keep in mind that for the *in vivo* use of the cement, the powder and the liquid must have been previously sterilised. In addition, a sterile working area is required to avoid contamination of the reagents at the time of preparation and introduction of the paste.

### Detailed explanation of the invention

The present invention introduces a thermosensitive inorganic cement for bone and/or dental applications, both in medicine and veterinary medicine. The cement in question has several specific properties that make it of special interest. In order to enhance said properties, the preparation conditions of the material have been optimised, as detailed below.

First of all, the cement object of this invention possesses thermosensitive properties that have not been previously described in any other cement formulations for biomedical use of calcium or magnesium phosphate, or calcium carbonate or sulphate. In other words, certain properties of the cement paste, such as injection force, cohesion and setting time, can be adjusted by controlling the paste temperature. This is achieved without compromising the biocompatibility of the material by means of the use of a poloxamer as an additive in the cement formulation.

Secondly, even with low liquid content in the paste, and unlike other fully injectable cements incorporating hydrogels in their liquid fraction, the injection force of the cement introduced in this invention is relatively low (∼50 N) and easy to reach by injecting by hand. This is achieved because the injection force diminishes as the temperature of the paste decreases. This property allows the surgeon to inject the material as accurately as possible and with a minimum of effort. Working temperatures to attain the lowest injection forces are temperatures slightly below the room temperature of an operating theatre (preferably between -5 and 18°C), which can be easily attained with the use of cooling systems, thermal baths, refrigerators or other similar or equivalent devices, easily accessible in hospitals, clinics or consulting rooms.

Thirdly, it is important to note that the cohesion of the pastes of the cement herein introduced increases with the increase in temperature of the medium where they are injected. It is of particular interest that, regardless of the initial temperature, the pastes of the cement herein introduced are cohesive just after being mixed and injected into a medium at a physiological temperature (37°C). This ensures that, once the cement paste is implanted in the patient's body, it will not disintegrate, but will keep its shape and harden *in vivo*, guaranteeing the success of the surgical procedure. Fourthly, the cement setting time is acceptable for clinical applications, giving the surgeon enough time to implant the cement with good injectability of the paste, and it hardens quickly once implanted. The hardening of the cement is due to two processes acting in parallel: one is the setting reaction of the cement and the other is the gelling of the poloxamer with the temperature increase due to body heat.

Fifthly, the addition of poloxamer does not affect the product of the setting reaction of the cement, which, in the case of a calcium phosphate cement, and depending on the specific composition of the cement, can be calcium-deficient hydroxyapatite, brushite, monetite or another calcium phosphate. In particular, these phosphates are similar to the mineral fraction of bones and teeth, which makes calcium phosphate cements biocompatible, osteoconductive and resorbable, and, as a result, they possess a large potential to regenerate lesions in hard tissues, bones and teeth in any vertebrates. On the other hand, in the case of other inorganic bone cements, the setting product comprises magnesium phosphates, calcium carbonates, calcium sulphates or mixtures thereof. The setting product can be both crystalline and amorphous.

Sixthly, the mechanical strength of the hardened cement may get to be higher than that of trabecular bone. In particular, optimised cements have a strength similar to those of several commercially available inorganic bone cements (compressive strength of about 30 MPa).

The preparation method of the cement object of the present patent requires a powder composed of one or several inorganic salts capable of hardening by a setting reaction upon contact with the liquid fraction. The liquid fraction is preferably composed of water, poloxamer in solution, and may or may not incorporate, as necessary, an additive to accelerate or delay the setting reaction. In addition to water, other biocompatible liquids may also be used where the poloxamer is soluble and the cement is capable of setting. Since the particle size distribution of the cement powder is one parameter controlling the injectability of the pastes, particle size control is a key factor of this invention. The optimal particle size in order to improve injectability without delaying setting or impairing the mechanical strength of the cement can be preferably achieved by milling the powder components, both in dry and wet conditions, using a water-free liquid, such as, for instance, ethanol or acetone, among others. The thermosensitive character of the cement is attained by adding a poloxamer in solution in the liquid fraction or in powder form in the solid fraction of the cement. Any variety of poloxamers can be used, depending on the particular properties one may want to attain. The mixing of the powder with the liquid fraction can be carried out at several liquid-powder ratios, although low ratios are preferred in order to improve the mechanical strength of the hardened cement, and at several mixing temperatures, preferably temperatures below room temperature, so as to be able to add a larger amount of powder in the poloxamer solution. It is recommended that the temperatures of the solid fraction and/or the liquid fraction before mixing be below 25°C and, preferably, below 12°C. The mixing can be carried out by hand or with a mechanical device of any type. In order to obtain smoother pastes in as little time as possible, a mechanical high-energy mixing device, of the planetary mixer type, is preferred. The paste obtained can be directly implanted in the bone or dental lesion to be treated, or it can be placed in a syringe or a similar device for its subsequent implantation by injection. Alternatively, once the paste has been placed in a syringe or a similar device, it may be frozen, preferably at temperatures equal to or below -20°C, for storage and subsequent thawing, preferably at less than 12°C for its use in clinical application.

Thermosensitive inorganic cements are of interest for bone and dental applications, both in medicine and veterinary medicine, especially in situations where it is necessary to increase the bone mass or to fill in a bone or dental cavity. Said cements can be used to fill in bone cavities, cranial defects or cavities left after the removal of a tumour, to fill in gaps after a multiple fracture, to fill in sockets after a tooth is extracted, to increase the amount of bone before placing a dental implant, to fix screws when placing metal plates, to attach fracture reduction fixtures, to stabilise bone fractures or to coat prostheses and implants, among others. The cement may likewise act as a support for a controlled-release drug delivery system or as a substrate for cellular ingrowth in tissue engineering scaffolds.

### Preparation of a thermosensitive inorganic cement for biomedical use

In order to illustrate the invention in a practical way, the possible reagents for the preparation of injectable thermosensitive inorganic cements for biomedical use are indicated below. Possibilities, however, are not limited to these examples. In the case of calcium phosphate cements, the powder fraction of the cement comprises alpha tricalcium phosphate, mixtures of tetracalcium phosphate with anhydride or dihydrated dicalcium phosphate, or mixtures of beta tricalcium phosphate with anhydride or hydrated monocalcium phosphate. For magnesium phosphate cements, the powder fraction of the cement comprises mixtures of magnesium oxide with one phosphate salt, such as sodium, potassium or ammonium phosphates or their mixtures. For calcium carbonate cements, the powder fraction of the cement comprises vaterite and/or amorphous calcium carbonate. For calcium sulphate cements, the powder fraction of the cement comprises hemihydrated calcium sulphate. In all mentioned cases, the powder fraction is preferably at a temperature below 25°C and, more preferably, below 12°C.

As the main component of the liquid fraction, distilled or bidistilled water is preferably used. Alternatively, water, saline aqueous solutions, simulated physiological fluids or other liquids can be used wherein the poloxamer is soluble and the cement is capable of setting. The poloxamer to be used may be any of the existing poloxamers, although those having high molecular weight are preferred, without implying any limitations. The poloxamer can be added as a fine ground powder to the cement powder, although its addition as a solution in the liquid fraction of the cement is preferred. In order to boost the thermosensitive effect of the cement, the use of poloxamers at such a concentration that the gelling temperature of the solution is close to room temperature, such as, for instance, poloxamer 407 between 10 and 30% by weight, is preferred. The temperature of the liquid fraction at the time of mixing ought to be below 25°C and, preferably, below 12°C.

In order to get a fully injectable paste, as well as the maximal mechanical strength of the cement, it is necessary to control the particle size of the reagents that make up the powder and the mixing protocol with the liquid fraction. As for particle size, optimum size may be achieved by milling the powder, both dry and wet, in the latter case using a water-free liquid, such as, for instance, ethanol or acetone, among others. The particle size distribution of the powder may vary between 0.1 and 80 µm, but should preferably be smaller than 20 µm. Alternatively, a separation by particle size can be carried out after milling so as to achieve the desired size distribution. The mixing of the powder with the liquid fraction can be carried out at different liquid-powder ratios, between 0.15 and 1.0 mL/g. Preferably, it is recommended that liquid-powder ratios between 0.2 and 0.4 mL/g be used so as to improve the mechanical strength of the cement. The mixing can be carried out by hand or with a mechanical device of any type. In order to obtain smoother pastes in as little time as possible, a mechanical high-energy mixing device, of the planetary mixer type, is preferred. It is recommended that the temperatures of the solid fraction and/or the liquid fraction before mixing be below 25°C and, more preferably, below 12°C. It is recommended that the mixing temperature preferably be between -5 and 20°C. Under these conditions, a paste with workable consistency is obtained that can be directly implanted in the bone or dental lesion to be treated, or it can be placed in a syringe or a similar device for its subsequent implantation by injection. The time elapsed between the preparation and the implantation of the paste depends on the specific cement formulation and it can vary between 5 and 60 minutes.

As for the thermosensitive character of the material and in order to minimise the force necessary to inject the cement, the temperature of the paste at the time of the injection should be below 25°C. It is recommended that the paste preferably be between -5 and 20°C. Any method available to cool the paste may be used. For instance, with no limitation of alternatives, cooling systems, thermal baths, refrigerators or other similar or equivalent devices, can be used.

Once placed inside a syringe or a similar device, the paste can also be frozen, preferably at temperatures equal to or below -20°C, for storage, and it is subsequently thawed preferably at less than 12°C for its use in clinical application.

It is important to keep in mind that for the *in vivo* use of the cement, the powder and the liquid must have been previously sterilised. In addition, a sterile working area is required to avoid contamination of the reagents at the time of preparation and introduction of the paste.

### Preferred embodiments

The examples included below are described in order to illustrate the invention in a practical way, with no intention to limit or restrict the specific compositions taken up therein and the preparation method for the mixture of ingredients.

### Example 1-Thermosensitive calcium phosphate cement: Control of the force of injection

150 g of alpha tricalcium phosphate were milled in an agate jar using a planetary mill and the following three milling stages: 1) 450 rpm for 60 minutes with 10 agate balls of 30 mm of diameter; 2) 500 rpm for 40 minutes with 10 agate balls of 30 mm of diameter; 3) 500 rpm for 60 minutes with 100 agate balls of 10 mm of diameter. 2.5 g of Na₂HPO₄ and 20 g of poloxamer 407 were dissolved in a total volume of 100 mL of distilled water. The dissolution of the components was carried out in a planetary high-energy mixer for 30 minutes at 3000 rpm using water at 4°C. The preparation of the paste consisted in mixing 5.714 g of alpha tricalcium phosphate powder with 2 ml of the Na₂HPO₄ and poloxamer 407 dissolution previously cooled down to 0°C. The mixing was carried out in a planetary high-energy mixer for 20 seconds at 1650 rpm.

The paste obtained was placed in a syringe of 5 mL of capacity and with an opening of 2 mm of diameter. Before injecting the cement paste, the syringes were submerged in a water bath at different temperatures for 15 minutes, so that the temperature of the paste at the time of injection was 0, 7, 12, 15, 18 or 20°C. The syringes at said temperatures were placed in a holder to carry out the injection test. This test consists in measuring the necessary force to inject the paste at a syringe plunger feed rate of 15 mm/min. The test was performed in a universal mechanical test machine (MTS Bionix 858). The test result is the injectability curve, which relates the force required for injecting the paste at a constant rate as a function of the plunger displacement, the latter being expressed as a percentage relative to the maximum possible stroke.

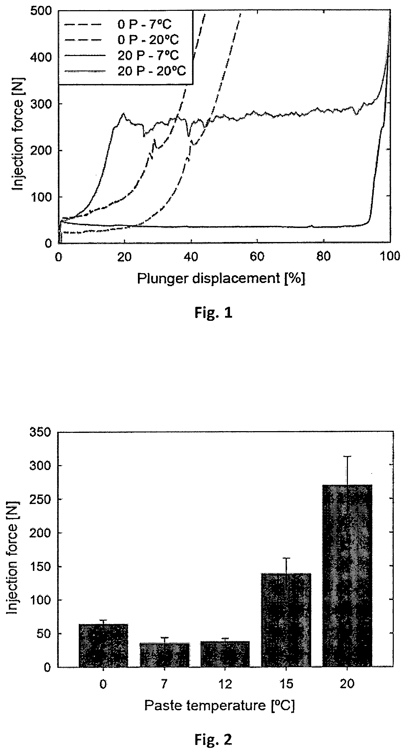

Figure 1 shows injection curves for cement paste containing poloxamer 407 at 7 and 20°C. Figure 1 also shows injection curves for cement paste without poloxamer 407 at the same temperatures. Without poloxamer 407, the injection force did not change considerably between 7 and 20°C (approximate value from 20 to 50 MPa). Without poloxamer 407, the paste was partially injected, since the plunger reached only between 45% and 55% of the possible stroke. The reason is the phenomenon known as fraction separation or filtering by pressure, which consists in that the liquid is injected through the powder, so that the solid fraction inside the syringe increases during the test. The increase of the solid fraction inside the syringe makes it increasingly difficult to inject the paste, requiring more force for the injection. With poloxamer 407, however, regardless of the temperature, the cement paste was injected in full, since the plunger reached the maximum possible stroke and no remaining paste was visually observed inside the syringes. It is interesting to note that, although in all cases the pastes were injected in full, the force required to inject them was significantly different between 7 and 20°C.

Figure 2 summarises the results of the force required to inject the thermosensitive cement pastes at different temperatures (0, 7, 12, 15, 18 and 20°C). The values are presented as the mean and standard deviation of at least three tests. Figure 2 shows that the force required to inject the paste diminishes as the temperature decreases. It is also observed that, for paste temperatures equal to or lower than 12°C, the force for the injection is minimal, taking values between 30 and 60 MPa. The cement pastes are fully injectable at all temperatures, and in no case do they exhibit any signs of fraction separation.

### Example 2-Thermosensitive calcium phosphate cement: Paste cohesion

150 g of alpha tricalcium phosphate were milled in an agate jar using a planetary mill and the following three milling stages: 1) 450 rpm for 60 minutes with 10 agate balls of 30 mm of diameter; 2) 500 rpm for 40 minutes with 10 agate balls of 30 mm of diameter; 3) 500 rpm for 60 minutes with 100 agate balls of 10 mm of diameter. 2.5 g of Na₂HPO₄ and 20 g of poloxamer 407 were dissolved in a total volume of 100 mL of distilled water. The dissolution of the components was carried out in a planetary high-energy mixer for 30 minutes at 3000 rpm using water at 4°C. The preparation of the paste consisted in mixing 5.714 or 3.636 g of alpha tricalcium phosphate powder with 2 ml of the Na₂HPO₄ and poloxamer 407 dissolution previously cooled down to 0°C. The mixing was carried out in a planetary high-energy mixer for 20 seconds at 1650 rpm.

The paste obtained was immediately placed in a syringe of 5 mL of capacity and with an opening of 2 mm of diameter. The syringes were submerged in a water bath at different temperatures for 15 minutes, so that the temperature of the paste was 0, 7 or 18°C. Once the paste reached the desired temperature, it was injected by hand into Ringer's solution (0.9% by weight of NaCl in water) at different temperatures between 0 and 37°C. Paste cohesion was visually determined for a maximum of 24 h, enough time for the hardening of the cement.

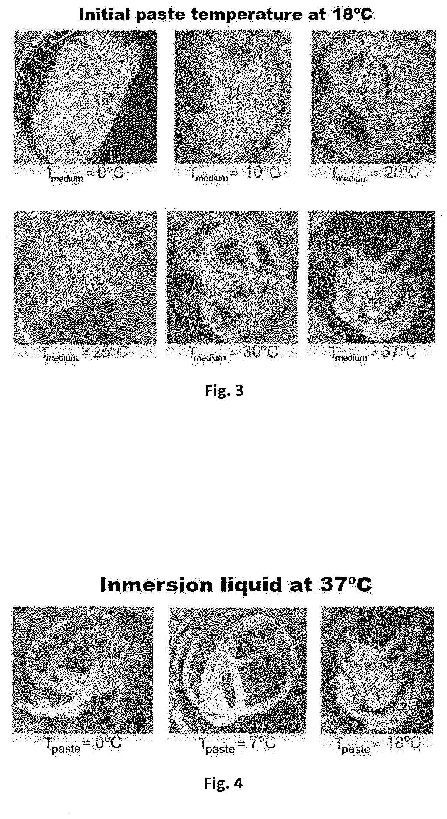

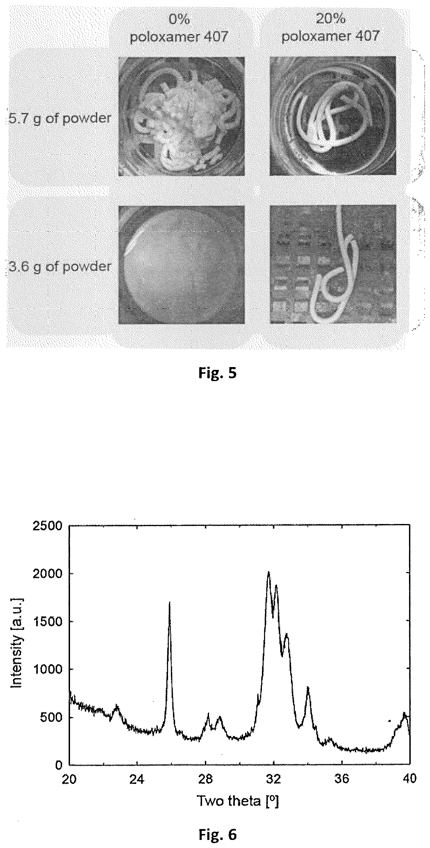

Figure 3 shows images of the paste initially at 18°C and injected into Ringer's solution at temperatures between 0 and 37°C. It can be observed that, as the temperature of Ringer's solution increases, the injected paste retains its extrusion form to a larger extent, which indicates that the paste cohesion increases with the temperature of the medium where it is injected. In particular, at 37°C (physiological temperature), form retention of the cement is complete, so it can be injected with the assurance that the cement will retain its integrity *in situ.* Figure 4 shows that the initial temperature of the paste (0, 7 or 18°C) does not affect cement cohesion when it is injected into Ringer's solution at 37°C. Figure 5 shows that cements without poloxamer 407 have no cohesion in Ringer's solution at 37°C. On the one hand, when a small amount of powder (3.636 g) is mixed with 2 mL of Na₂HPO₄ solution at 2% by weight, the paste is so liquid that it disintegrates immediately upon contact with Ringer's solution. On the other hand, when the amount of powder is higher (5.714 g), the paste seems to have cohesion after the first few hours in contact with Ringer's solution, but with the passage of time the extrusion form loses continuity, giving rise to several microscopic fragments that, even though they end up hardening, do not maintain their original shape. With poloxamer 407, however, cement pastes harden, fully maintaining their form for the two amounts of powder used.

### Example 3-Setting and hardening of thermosensitive calcium phosphate cement

150 g of alpha tricalcium phosphate were milled in an agate jar using a planetary mill and the following three milling stages: 1) 450 rpm for 60 minutes with 10 agate balls of 30 mm of diameter; 2) 500 rpm for 40 minutes with 10 agate balls of 30 mm of diameter; 3) 500 rpm for 60 minutes with 100 agate balls of 10 mm of diameter. 2.5 g of Na₂HPO₄ and 20 g of poloxamer 407 were dissolved in a total volume of 100 mL of distilled water. The dissolution of the components was carried out in a planetary high-energy mixer for 30 minutes at 3000 rpm using water at 4°C. The preparation of the paste consisted in mixing 5.714 g of alpha tricalcium phosphate powder with 2 ml of the Na₂HPO₄ and poloxamer 407 dissolution previously cooled down to 0°C. The mixing was carried out in a planetary high-energy mixer for 20 seconds at 1650 rpm.

The initial and final setting times were measured by means of the Gillmore needle test, according to which initial setting is defined as the time elapsed from the moment the powder contacts the liquid until a pressure of 0.3 MPa leaves no marks on the surface of the cement, whereas the final setting time is defined as the time elapsed from the moment the powder contacts the liquid until a pressure of 5 MPa leaves no marks on the surface of the cement. The test was performed placing the cement paste in cylindrical plastic moulds 10 mm tall. The initial setting time of the cement was 11.6 minutes and the final setting time was 33.7 minutes.

In order to determine the final product of the setting reaction and the compressive strength of the cement, samples of the cement were prepared in test tubes of 12 mm in height and 6 mm of diameter in Teflon moulds. Said moulds were placed in Ringer's solution (0.9% by weight of NaCl in water) and held at 37°C for 7 days. Figure 6 shows the X-ray diffraction pattern of the hardened cement for 7 days. The observed diffraction maxima coincide with the crystallographic pattern of hydroxyapatite (JCPDS 09-0342). This indicates that, during setting, alpha tricalcium phosphate hydrolyses into hydroxyapatite. In particular, and according to the Ca/P ratio of the alpha tricalcium phosphate used as a reagent (Ca/P = 1.5), the hydroxyapatite formed corresponds to a calcium-deficient hydroxyapatite. Compressive strength was measured by means of a universal mechanical test machine with a 10 kN load cell, using a jaw displacement speed of 1mm/min. The compressive strength of the hardened thermosensitive cement was 28.4 ± 5.1 MPa. The skeletal density of the hardened thermosensitive cement measured by helium pycnometry was 2.68 g/cm³.

### Example 4-Low-temperature storage of cement paste: Freezing and thawing

150 g of alpha tricalcium phosphate were milled in an agate jar using a planetary mill and the following three milling stages: 1) 450 rpm for 60 minutes with 10 agate balls of 30 mm of diameter; 2) 500 rpm for 40 minutes with 10 agate balls of 30 mm of diameter; 3) 500 rpm for 60 minutes with 100 agate balls of 10 mm of diameter. 2.5 g of Na₂HPO₄ and 20 g of poloxamer 407 were dissolved in a total volume of 100 mL of distilled water. The dissolution of the components was carried out in a planetary high-energy mixer for 30 minutes at 3000 rpm using water at 4°C. The preparation of the paste consisted in mixing 5.714 g of alpha tricalcium phosphate powder with 2 ml of the Na₂HPO₄ and poloxamer 407 dissolution previously cooled down to 0°C. The mixing was carried out in a planetary high-energy mixer for 20 seconds at 1650 rpm.

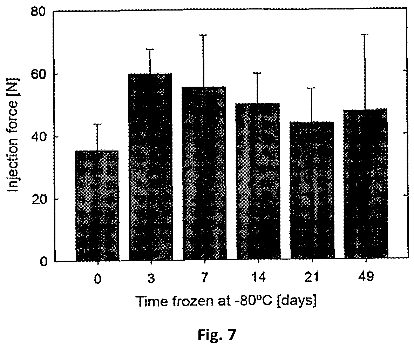

The paste obtained was immediately placed in a syringe of 5 mL of capacity and with an opening of 2 mm of diameter. The syringes were submerged in liquid nitrogen for 15 minutes and immediately placed at -80°C in a freezer for 3, 7, 14, 21 and 49 days. After these times, the syringes with the cement paste were thawed for 13 minutes in a water bath at 7°C. The force required to inject the thawed cement paste was determined by means of the injection test. The test was performed in a universal mechanical test machine (MTS Bionix 858) at a syringe plunger feed rate of 15 mm/min. Figure 7 shows that the force required to inject the thawed cement pastes did not significantly increase (p > 0.05) with respect to the force required to inject the freshly prepared paste at the same temperature. In all cases, the paste was injected in full, since the plunger reached the end of its stroke and no remaining paste was observed inside the syringes. The thawed pastes exhibited thorough cohesion when they were injected into Ringer's solution (0.9% by weight of NaCl in water) at 37°C. After the various freezing times, the initial and final setting times, measured by means of the Gillmore needle test, did not change significantly (p > 0.05) with respect to the initial and final setting times (respectively) of non-frozen pastes. Neither were any statistically significant changes (p > 0.05) found in the compressive strength of the cement hardened for 7 days due to the freezing and thawing of the cement paste.

### Brief description of the drawings

In the Figures, the following has been depicted:
**Figure 1****.** Injection curves (force - plunger displacement) corresponding to cements with poloxamer 407 (solid lines) at 7 and 20°C, and without poloxamer 407 (broken lines) at 7 and 20°C.
**Figure 2****.** Forces required to inject the thermosensitive cement pastes at different temperatures (0, 7, 12, 15, 18 and 20°C).
**Figure 3****.** Images of the cohesion of the thermosensitive cement pastes in Ringer's solution at temperatures between 0 and 37°C. In all cases, the initial temperature of the paste was 18°C.
**Figure 4****.** Images of the cohesion of the thermosensitive cement pastes initially held at 0, 7 or 18°C, upon injection into Ringer's solution at 37°C.
**Figure 5****.** Images of the cohesion of the cement pastes with different amounts of powder and 2 mL of liquid fraction, with and without the addition of poloxamer 407.
**Figure 6****.** X-ray diffraction of the reaction product of a hardened thermosensitive cement in Ringer's solution for 7 days at 37°C.
**Figure 7****.** Forces required to inject the thermosensitive cement pastes after 0, 3, 7, 14, 21 and 49 days of freezing at -80°C. In all cases, the pastes were thawed and then injected at 7°C.

### Scaling the invention up to an industrial application

The thermosensitive inorganic cements that have been described have several applications in the clinical and veterinary realms, especially in the field of orthopaedic surgery, to fill in bone cavities, cranial defects or cavities left after the removal of a tumour, to fill in gaps after a fracture, to fix screws when placing metal plates, to stabilise bone fractures or to coat prostheses and implants, or, in dentistry, to fill in sockets after a tooth is extracted or to increase the amount of bone before placing a dental implant. This invention is readily applicable on an industrial scale. The preparation of the powder and the liquid fraction of the cements is very simple, since all the reagents are commercially available and the only thing required is optimising the powder particle size and the temperature of the paste for achieving the advantages listed below, which increase the value of said cement in the range of biomaterials.
(a) Minimising the force required to inject the paste, facilitating the implantation procedure by injection;
(b) Attaining total cohesion of the paste when it is injected at physiological temperature;
(c) Quick setting accompanied by high compressive strengths;
(d) Absence of toxic by-products;
(e) Adhesion to living tissues, such as bone;
(f) *In vivo* resorption.

Likewise, the preparation of the cement is simple. The use of the cement in biomedical applications requires the sterilisation of the reagents, as well as their proper use. In addition, the cement must be placed in the bone or dental defect in a short time, limited by its hardening.

### REFERENCES

[1] G. Dumortier, J. L. Grossiord, F. Agnely, and J. C. Chaumeil, "A review of poloxamer 407 pharmaceutical and pharmacological characteristics," Pharm. Res., vol. 23, no. 12, pp. 2709-28, Dec. 2006.
[2] J. Franco, P. Hunger, M. E. Launey, A. P. Tomsia, and E. Saiz, "Direct write assembly of calcium phosphate scaffolds using a water-based hydrogel," Acta Biomater., vol. 6, no. 1, pp. 218-28, Jan. 2010.
[3] J. Raymond and A. Schwarz, "Temporary embolization using inverse thermosensitive polymers," *US Pat. App.* 10/794,804, 2004.
[4] D. O'mahony and V. Garigapati, "Post irradiation shelf-stable dual paste direct injectable bone cement precursor systems and methods of making same," *US Pat. App.* 13/ ..., 2013.
[5] M. P. Ginebra, J. A. Planell, and F. X. Gil, "Injectable, Self Setting Calcium Phosphate Foam," EP20050782615 200508112007.

## Claims

1. A thermosensitive inorganic cement for biomedical use **characterised in that** it comprises one inorganic hydraulic cement and one poloxamer, which can be added either in the solid fraction or in the liquid fraction of the inorganic hydraulic cement, said solid fraction of the inorganic hydraulic cement comprising calcium, sodium and/or ammonium phosphates, calcium carbonates, calcium sulphates, magnesium oxide or any mixture thereof, and the liquid fraction of the inorganic hydraulic cement is water or an aqueous solution.

2. A cement according to claim 1 wherein the poloxamer is preferably added in the liquid fraction of the cement, which comprises distilled water or an aqueous solution.

3. A cement according to claim 1 wherein the self-setting component is alpha tricalcium phosphate.

4. A cement according to claim 1 wherein the poloxamer used as an additive is poloxamer 407, **characterised in that** the poloxamer 407 can be added as a powder in the solid fraction of the cement or preferably dissolved in the liquid fraction of the cement.

5. A cement according to claim 4 wherein the poloxamer 407 is added in the liquid fraction of the cement in a concentration between 10 and 30% by weight, preferably 20% by weight.

6. A cement according to claims 3 to 5 wherein the liquid fraction of the cement is preferably an aqueous solution of Na₂HPO₄, with a concentration of less than 10% by weight, preferably less than 2.5% by weight.

7. A cement according to claims 3 to 5 wherein the mixture of the liquid fraction with the solid fraction is carried out at a liquid-powder ratio between 0.15 and 1.0 mL/g, preferably between 0.2 and 0.4 mL/g.

8. A method for the preparation of the thermosensitive inorganic cement for biomedical applications wherein the liquid fraction of the inorganic hydraulic cement, formed by a poloxamer hydrogel, at a temperature below 25°C and preferably below 12°C, is mixed with the powder fraction of the cement, which comprises calcium, sodium and/or ammonium phosphates, calcium carbonates, calcium sulphates, magnesium oxide or any mixture thereof.

9. A method for the preparation of a thermosensitive inorganic cement for biomedical applications according to claim 8 wherein the powder fraction is at a temperature preferably below 25°C, and more preferably below 12°C.

10. A method for the preparation of a thermosensitive inorganic cement for biomedical applications according to claims 8 and 9 wherein the powder fraction preferably comprises alpha tricalcium phosphate.

11. A method for the preparation of a thermosensitive inorganic cement for biomedical applications according to claim 10 wherein the mixture of the liquid fraction with the solid fraction is carried out at a liquid-powder ratio between 0.15 and 1.0 mL/g, preferably between 0.2 and 0.4 mL/g.

12. A method for the preparation of the thermosensitive inorganic cement for biomedical applications according to claim 8 wherein the mixture of the powder with the liquid fraction is preferably carried out in a high-energy mixer.

13. A method for the preparation of the thermosensitive inorganic cement for biomedical applications according to claim 8 wherein, once placed inside a syringe or a similar device, the paste is frozen, preferably at temperatures equal to or below -20°C, for storage, and it is subsequently thawed preferably at less than 12°C for use in clinical application.
